# EUROPÄISCHE PATENTSCHRIFT

(11) **EP 0 518 021 B1**
(45) Veröffentlichungstag und Bekanntmachung des Hinweises auf die Patenterteilung: **20.08.1997**
(21) Anmeldenummer: 92106196.6
(22) Anmeldetag: 10.04.1992
(51) Int. Cl.: C07C 2/30, B01J 31/14, C07C 2/38, C07C 11/21

(54) **Verfahren zur Herstellung von Methylidengruppen enthaltenden Alpha-Omega-ungesättigten Oligomeren aus Alpha, Omega-Diolefinen in Gegenwart von aluminiumorganischen Verbindungen als Katalysator**
Method for the production of alpha, omega-unsaturated oligomers containing methylidene groups from alpha, omega-diolefines in the presence of organoaluminium compounds as catalyst
Procédé pour la fabrication d'oligomères alpha, oméga-insaturés contenant des groupements méthylidènes à partir de dioléfines-alpha, oméga en présence de composés aluminiumorganiques comme catalyseur

(30) Priorität: 12.06.1991 DE 4119332
(43) Veröffentlichungstag der Anmeldung: 16.12.1992
(73) Patentinhaber: RÖHM GMBH, 64293 Darmstadt (DE)
(72) Erfinder: Streck, Roland, Dr., W-4370 Marl 1 (DE); Monkiewicz, Jaroslaw, Dr., W-4370 Marl 1 (DE); Wey, Hans Günther, Dr., W-4330 Mühlheim/Ruhr (DE)

(56) Entgegenhaltungen:
- US-A- 3 088 985
- US-A- 3 391 209
- US-A- 3 522 321
- JOURNAL OF ORGANIC CHEMISTRY., Bd.28, Nr.11, 1963, EASTON US Seiten 3237 - 3238 G. HATA ET AL

## Beschreibung

Die Erfindung bezieht sich auf ein Verfahren zur Herstellung von Methylidengruppen enthaltenden α,ω-ungesättigten Oligomeren aus α,ω-Diolefinen in Gegenwart von aluminiumorganischen Verbindungen als Katalysator sowie auf die nach diesem Verfahren hergestellten Stoffe.

Es ist bekannt, daß α-Olefine beim Erhitzen mit katalytischen Mengen an Aluminiumtrialkylen oder -dialkylhydriden zu Gemischen reagieren, in denen weit überwiegend β-verzweigte α-Olefine doppelter Kohlenstoff-Zahl des Ausgangsolefins vorkommen: So entsteht beispielsweise bei der Dimerisierung von Propen 2-Methyl-1-penten, bzw. von 1-Buten das 2-Ethyl-1-hexen als Hauptprodukt (siehe DE-PSS 878 560 und 925 291 sowie in Liebigs Ann. Chem. 629 (1960) 121 - 166).

Bei der Übertragung dieser Reaktion auf Kohlenwasserstoffe mit zwei endständigen Vinylgruppen (α,ω-Diolefine) wurden bislang hauptsächlich cyclische Produkte isoliert. So entsteht aus Butadien ein Gemisch von Methyldimethylencyclopentanen (US-PSS 3 356 704 und 3 391 209, J. Org. Chem. 34 (1969) 1931 - 1935), während mit 1,5-Hexadien sowie mit 1,6-Heptadien eine Cyclisierung zum Methylencyclopentan bzw. zum Methylencyclohexan stattfindet (Angew. Chem. 70 (1967) 862 - 863 und Tetrahedron Lett. 1976, 1257 - 1258). Die gleiche Beobachtung wurde auch bei Versuchen zur Bis-Hydroaluminierung von α,ω-Diolefinen gemacht (J. Org. Chem. 28 (1963) 3237 - 3238). Über die Entstehung von geringen Mengen nicht weiter beschriebenen höheren Homologen aus α,ω-Diolefinen wurde bisher nur am Rande berichtet, und dieses war auch nicht ausdrückliches Ziel dieser Synthesen. Verfahren zur Herstellung von polymeren Kohlenwasserstoffen ausgehend von α,ω-Diolefinen mit aluminiumorganischen Verbindungen als Katalysator sind nicht bekannt.

Die vorliegende Erfindung geht daher von der Aufgabenstellung aus, weitgehend lineare, höhermolekulare Kohlenwasserstoffe mit reaktiven Doppelbindungen aus α,ω-Diolefinen herzustellen.

Die Aufgabe wird dadurch gelöst, daß α,ω-Diolefine der allgemeinen Formel 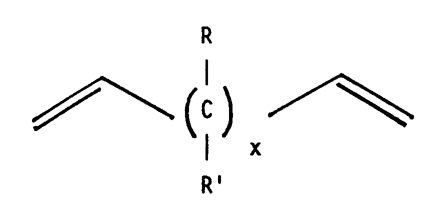 in flüssiger Phase bei einer Temperatur von 150 bis 350 °C unter Zusatz von katalytisch wirkenden Mengen an aluminiumorganischen Verbindungen der allgemeinen Formel AlX₃ oder AlX₂H zur Reaktion gebracht werden.

Typische α, -Diolefine, die sich zur Synthese der erfindungsgemäßen Oligomeren eignen, sind 1,4-Pentadien, 1,6-Heptadien, 1,7-Octadien, 1,8-Nonadien, 1,9-Decadien, 1,10-Undecadien, 1,11-Dodecadien, 1,12-Tridecadien, 1,13-Tetradecadien, 1,14-Pentadecadien, 1,15-Hexadecadien, 1,16-Heptadecadien, 1,17-Octadecadien, 1,18-Nonadecadien, 1,19-Eicosadien, 1,3- bzw. 1,4-Divinylcyclohexan, 1,3,5-Trivinylcyclohexan usw. sowie ihre, außer an den Doppelbindungen beliebig durch Alkyl-, Cycloalkyl-, Aryl- oder Aralkylgruppen mit 1 bis 30 Kohlenstoffatomen substituierten Derivate. Über die vorstehend genannten α, -Diolefine hinaus können auch höher ungesättigte Kohlenwasserstoffe zum Kettenaufbau verwendet werden. Unter den vorgegebenen Reaktionsbedingungen werden dann nur die endständigen Vinylgruppen umgesetzt. Obwohl eine solche Verbindung von der Gesamtzahl der Doppelbindungen her gerechnet ein Tri-, Tetra- oder gar Multiolefin ist, wirkt diese praktisch nur als bifunktioneller Kettenbaustein. Als Beispiele für solche Olefine seien genannt: 1,4,7-Octatrien, 1,4,7-, 1,4,8-Nonatrien, 1,4,9-, 1,5,9-Decatrien, 1,4,10-, 1,5,10-Undecatrien, 1,4,7,11-, 1,5,8,11-, 1,4,8,11-, 1,5,7,11-Dodecatetraen, 1,5,9,13-Tetradecatetraen, 4-Methyl-1,4,7-octatrien, 5,6-Dimethyl-1,5,9-decatrien, 4,4-Dimethyl-6,7-diethyl-1,5,12-tridecatrien usw.

Als Katalysatoren können alle aluminiumorganischen Verbindungen verwendet werden, welche den Formeln AlX₃ oder AlX₂H entsprechen, wobei X ein beliebiger aliphatischer, alicyclischer oder aromatischer Kohlenwasserstoffrest ist, der 1 - 30 Kohlenstoffatome - vorzugsweise 2 - 20 Kohlenstoffatome - enthält. Darunter befinden sich beispielsweise: Aluminiumtrimethyl, Aluminiumtriethyl, Aluminiumtripropyl, Aluminiumtriisopropyl, Aluminiumtri-n-butyl, Aluminiumtri-sec.-butyl, Aluminiumtri-n-pentyl, Aluminiumtriisobutyl, Aluminiumtri-2-methylpentyl, Aluminiumtri-n-hexyl, Aluminium-trineohexyl, Aluminiumtri-n-heptyl, Aluminiumtri-n-octyl, Aluminiumtri-2-ethylhexyl, Aluminiumtricyclopentyl, Aluminiumtricyclohexyl, Aluminiumtriethylcyclohexenyl, Aluminiumtriphenyl, Aluminiumtriphenylethyl, Aluminiumtrinaphthyl, sowie die bei der sog. "Aufbau-Reaktion" durch Ethylenaufnahme aus niederen Aluminiumtrialkylen entstehenden Gemische höherer Aluminiumtrialkyle, sowie auch Dimethylaluminiumhydrid, Diethylaluminiumhydrid, Dipropylaluminiumhydrid, Di-n-butylaluminiumhydrid, Diisobutylaluminiumhydrid, Dipentylaluminiumhydrid, Dihexylaluminiumhydrid, Diheptylaluminiumhydrid, Dioctylaluminiumhydrid, Dinonylaluminiumhydrid und Didecylaluminiumhydrid usw.

Die katalytisch wirksamen Verbindungen können direkt einzeln oder als Mischungen zugesetzt werden. Man kann sie aber auch aus unwirksamen Vorstufen erst in Gegenwart der umzusetzenden ungesättigten Kohlenwasserstoffe herstellen, z. B. Aluminiumtriethyl aus Diethylaluminiumchlorid und metallischem Natrium bzw. Natriumaluminiumtetraethyl.

Die Konzentration des Katalysators kann in weiten Grenzen schwanken, wobei man im praktischen Fall meist bei einer Konzentration zwischen 0,1 und 10 Mol-%, bezogen auf die eingesetzte Stoffmenge Diolefin, bleiben wird. Besonders bevorzugt wird eine Katalysator-Konzentration von 0,1 bis 5 Mol-%.

Die Reaktionstemperaturen liegen zwischen 150 und 350 °C, vorzugsweise zwischen 180 und 250 °C. Die Reaktionszeiten nehmen mit steigender Katalysator-Konzentration ab und liegen in einem Bereich von 48 Stunden bis 5 Minuten. Die passende Kombination der drei Variabeln Katalysator-Konzentration, Reaktionstemperatur und Reaktionszeit ist so zu wählen, daß die Bildung von unerwünschten Nebenprodukten durch Isomerisierung der endständigen Vinylgruppe oder der Methylidengruppen weitgehend zurückgedrängt wird. Der Fachmann kann diese jedoch leicht durch einige orientierende Versuche und passende Inter- bzw. Extrapolationen finden.

Diskontinuierlich in Reaktoren (Autoklaven, Rührkessel) durchgeführte Reaktionen erfordern relativ lange Reaktionszeiten. Wird jedoch die Reaktion in kontinuierlich betriebenen Reaktoren mit kleinem Betriebsinhalt und engem Verweilspektrum - also z. B. Strömungsrohre - durchgeführt, so lassen sich Reaktionszeiten unterhalb einer Stunde bis zu wenigen Minuten realisieren.

Dies ist vor allem bei hohen Reaktionstemperaturen zu berücksichtigen, weil dann auch die Tendenz des Katalysators zum irreversiblen Zerfall unter Bildung von Aluminium, Aluminiumcarbid usw. erhöht ist.

Die Anwendung von Druck ist nur insofern erforderlich, als man das Verbleiben der Reaktanten in flüssiger Phase erzielen will. Man wird also zweckmäßigerweise unter dem Druck arbeiten, der sich als Dampfdruck der Reaktanten bei der gewünschten Arbeitstemperatur von selbst einstellt. Im Bereich der möglichen Reaktionstemperaturen von 150 - 350 °C können - abhängig von der Flüchtigkeit der jeweiligen Monomeren - dabei Drucke von 0,01 bis 200 bar auftreten.

Die Reaktion kann sowohl in Lösung als auch in Substanz durchgeführt werden, wobei Erniedrigung der Viskosität für die Verwendung von Lösemitteln sprechen kann, vereinfachter Materialeinsatz und Fortfall von Abtrennungs- und Rückführungsproblemen für die Massefahrweise.

Verwendet man Lösemittel, kommen nur trockene gesättigte bzw. aromatische trockene Kohlenwasserstoffe in Betracht. In diesem Sinne brauchbar sind u.a. folgende Lösemittel: n-Butan, Pentan und seine Isomeren, Hexan und seine Isomeren, Heptan und seine Isomeren, Octan und seine Isomeren, Nonan und seine Isomeren, Decan und seine Isomeren usw., Cyclopentan, Methylcyclopentan, Dimethylcyclopentan, Cyclohexan, Methylcyclohexan, Ethylcyclohexan, Dimethylcyclohexan, Cyclooctan, Methylcyclooctan, Dimethylcyclooctan, Ethylcyclooctan, Isopropylcyclohexan, Trimethylcyclododecan, Benzol, Toluol, o-, p-, m-Kresol, Cumol, Ethylbenzol, Dodecylbenzol (Isomerengemisch), Petrolether, Ligroin, Kerosin und ähnliche Erdölfraktionen, Paraffinöl, Tetralin, Decalin, Mono- und Dibenzyltoluol, Diphenyl.

Eine unabhängige Regelung des Molekulargewichtes kann durch Zusatz von monofunktionellen Olefinen - vornehmlich α-Olefinen - bewirkt werden, die gesättigte, also nicht zu weiterer Oligomerisierung fähige Endgruppen im Produkt bilden. Zwangsläufig entstehen bei dieser Molekulargewichtsregelung auch gewisse Mengen von Homodimeren des eingesetzten Monoolefins. Dies ist jedoch nur von untergeordneter Bedeutung, da diese Dimeren leicht aus dem Reaktionsgemisch abgetrennt werden können und für diese Stoffe ebenfalls nützliche Verwendungen existieren. Darüber hinaus führt auch die Anwesenheit von Wasserstoff (10 bis 250 bar) bei der Oligomerisierung zur Erniedrigung der Molekulargewichte der Produkte.

Die zur Molekulargewichtsregelung einsetzbaren Monoolefine sind prinzipiell alle ungesättigten Kohlenwasserstoffe mit einer Vinylgruppe, die mit Hilfe von Aluminiumtrialkylen bzw. -dialkylhydriden dimerisierbar sind, d.h. alle linearen und nicht an der Doppelbindung verzweigten α, -Olefine mit 3 bis 30 Kohlenstoffatomen, z.B. Propen, 1-Buten, 1-Penten, 3-Methyl-1-buten, 1-Hexen, 3-Methyl-1-penten, 4-Methyl-1-penten, 3,3-Dimethyl-1-buten, 1-Hepten, 3-Methyl-1-hexen, 4-Methyl-1-hexen, 5-Methyl-1-hexen, 3,4-Dimethyl-1-penten, 3,3-Dimethyl-1-penten, 1-Octen, 3-Methyl-1-hepten, 4-Methyl-1-hepten, 5-Methyl-1-hepten, 6-Methyl-1-hepten, 3,3-Dimethyl-1-hexen, 3,4-Dimethyl-1-hexen, 4,4-Dimethyl-1-hexen, 3,5-Dimethyl-1-hexen, 5,5-Dimethyl-1-hexen, 4,5-Dimethyl-1-hexen, 3-Ethyl-1-hexen, 4-Ethyl-1-hexen, 3,3,4-Trimethyl-1-penten, 3,4,4-Trimethyl-1-penten, 1-Nonen, 3-Methyl-1-octen, 4-Methyl-1-octen, 5-Methyl-1-octen, 6-Methyl-1-octen, 7-Methyl-1-octen usw. Auch Vinylgruppen tragende cyclische Kohlenwasserstoffe sind einsetzbar, wie z.B. Vinylcyclopentan, Vinylcyclohexan, Allylcyclopentan, Allylcyclohexan, 1-Butenylcyclohexan, 1-Pentencycloheptan, 1-Hexenylcyclooctan, 1-Octenylcycloundecan, Styrol, Allylbenzol, 1-Undecylnaphthaline, Vinylanthracene usw.

Es ist auch möglich, anstelle der α-Olefine solche mehrfach ungesättigten Kohlenwasserstoffe zu verwenden, die eine endständige unsubstituierte Doppelbindung und eine oder mehrere mittelständige und/oder substituierte Doppelbindungen besitzen. Beispiel für ein solches, nur teilweise reagierendes Diolefin ist 4-Vinylcyclohexen.

Überraschenderweise bilden sich keine Hochpolymeren, sondern die Molekulargewichte der Reaktionsprodukte bleibt auf den Oligomerbereich beschränkt, so daß ein zunächst nicht vorhergesehener Abbruchmechanismus angenommen werden muß.

Wie aus den Beispielen hervorgeht, werden erfindungsgemäß unter den gegebenen Bedingungen hauptsächlich solche Produkte erhalten, bei denen der Oligomerisierungsgrad (m) von über 70 % in einem Bereich von m gleich 3 bis 20 liegt.

Die erfindungsgemäßen Oligomere lassen sich z. B. nach Hydrocyanierung und Hydroformylierung und weiterer Oxidation oder Hydrierung als wertvolle Rohprodukte im Lack- und Klebstoffsektor verwenden.

Die nachfolgenden Beispiele dienen dem besseren Verständnis der Erfindung.

### Beispiele 1 - 14

In einem mit Stickstoff gespülten Stahlautoklaven werden die jeweiligen α,ω-Diolefine bzw. α,ω-Triolefine in Gegenwart des Katalysators und gegebenenfalls eines Lösemittels bei den angegebenen Reaktionstemperaturen und -zeiten bis zum Erhalt der jeweiligen Produkte umgesetzt. Anschließend wird die Mischung nach Abkühlen zunächst mit Methanol und danach mit 5%iger Natriumhydroxidlösung versetzt und ausgeschüttelt. Die wäßrige Phase wird abgetrennt und die organische Phase bis zur Neutralität mit destilliertem Wasser gewaschen. Nach Trocknen über Natriumsulfat werden zunächst Lösemittel und restliches Monomer bei Atmosphärendruck, danach die Dimeren im Vakuum destillativ abgetrennt. Das zurückbleibende Produkt wird durch Gelpermeationschromatographie und Bestimmung der Iodzahl charakterisiert. Alle weiteren Angaben über Ausgangs-, Endprodukte sowie Reaktionsbedingungen sind der Tabelle 1 zu entnehmen.

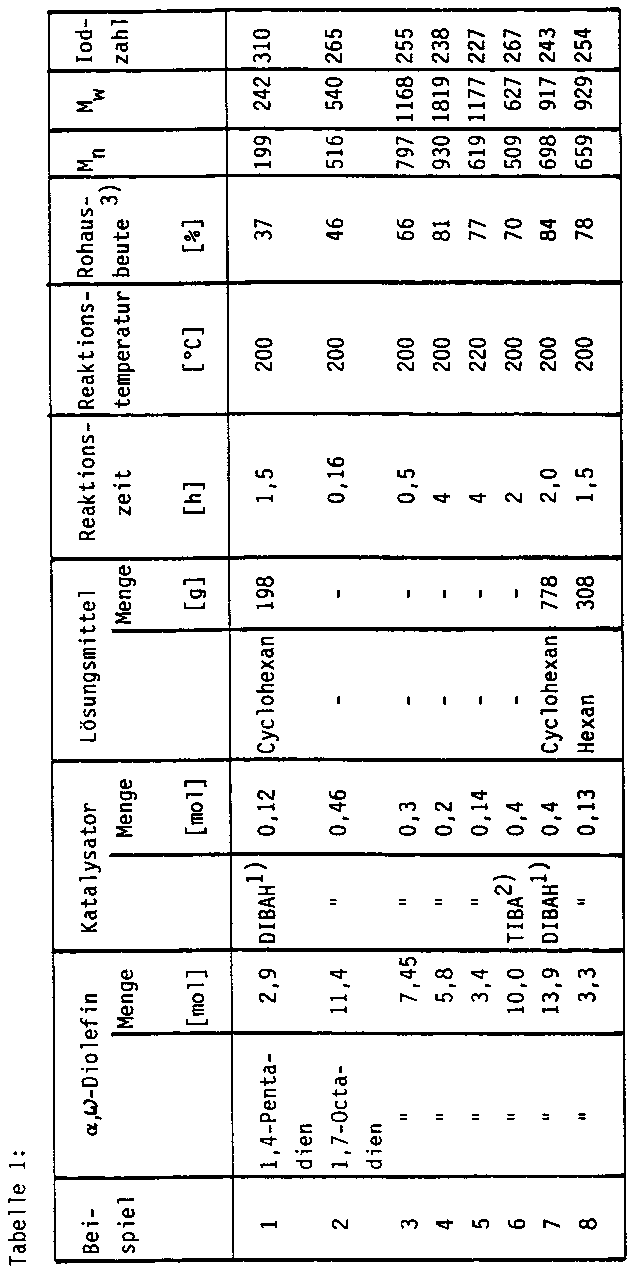

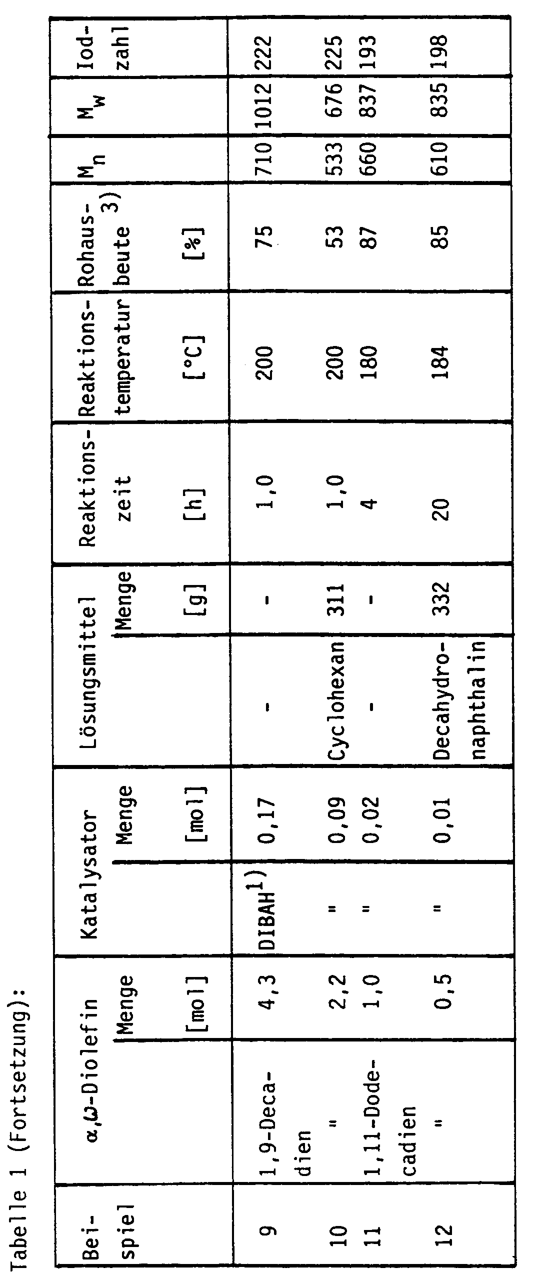

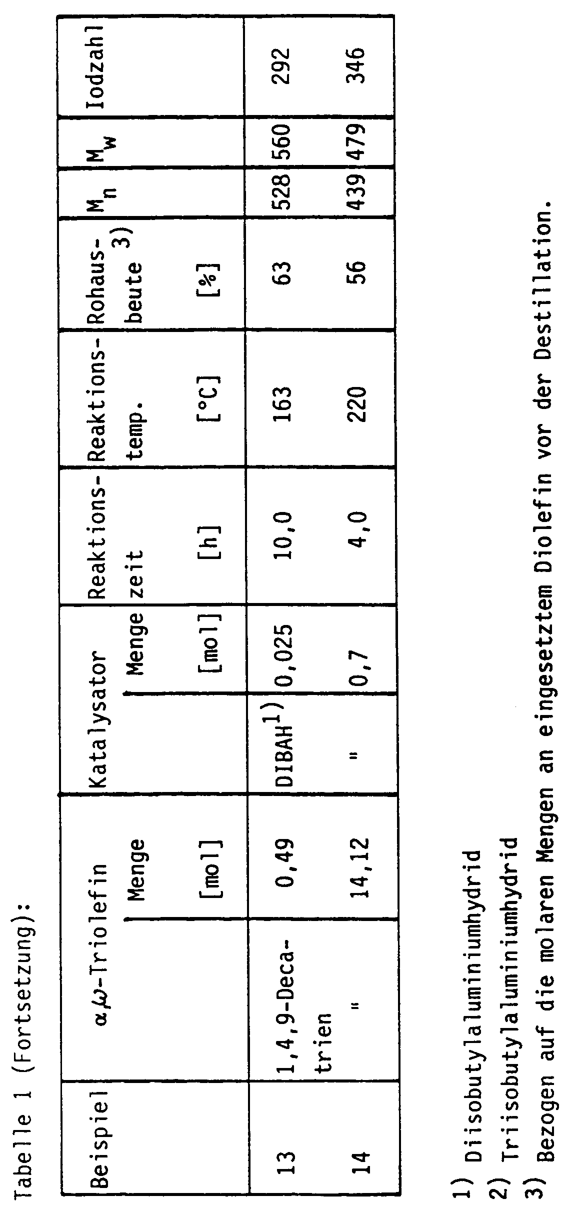

### Beispiele 15 - 21

In der gleichen Apparatur wird zunächst nach identischer Arbeitsweise wie in den Beispiele 1 bis 14 verfahren. Nach Ablauf einer angegebenen Reaktionszeit II wird die Apparatur bis zu einem Druck von 150 bar mit Wasserstoff gefüllt. Danach wird die Reaktion bis zum Erhalt der jeweiligen Endprodukte fortgeführt. Anschließend findet die Aufarbeitung der Mischung und die Charakterisierung der Produkte wie in den Beispiel 1 bis 14 statt. Alle weiteren Angaben über Ausgangs-, Endprodukte sowie Reaktionsbedingungen sind der Tabelle 2 zu entnehmen.

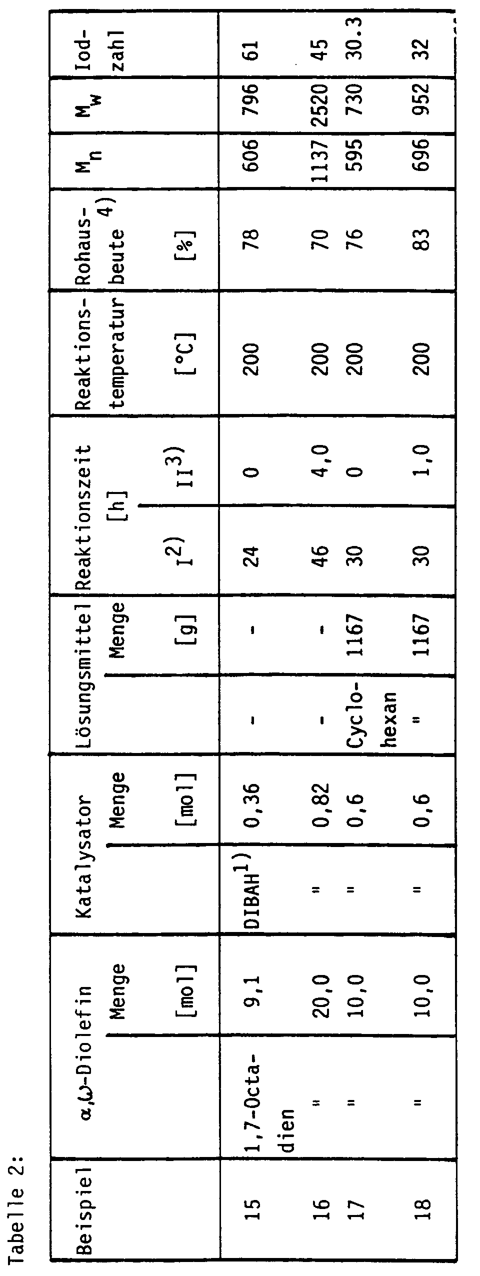

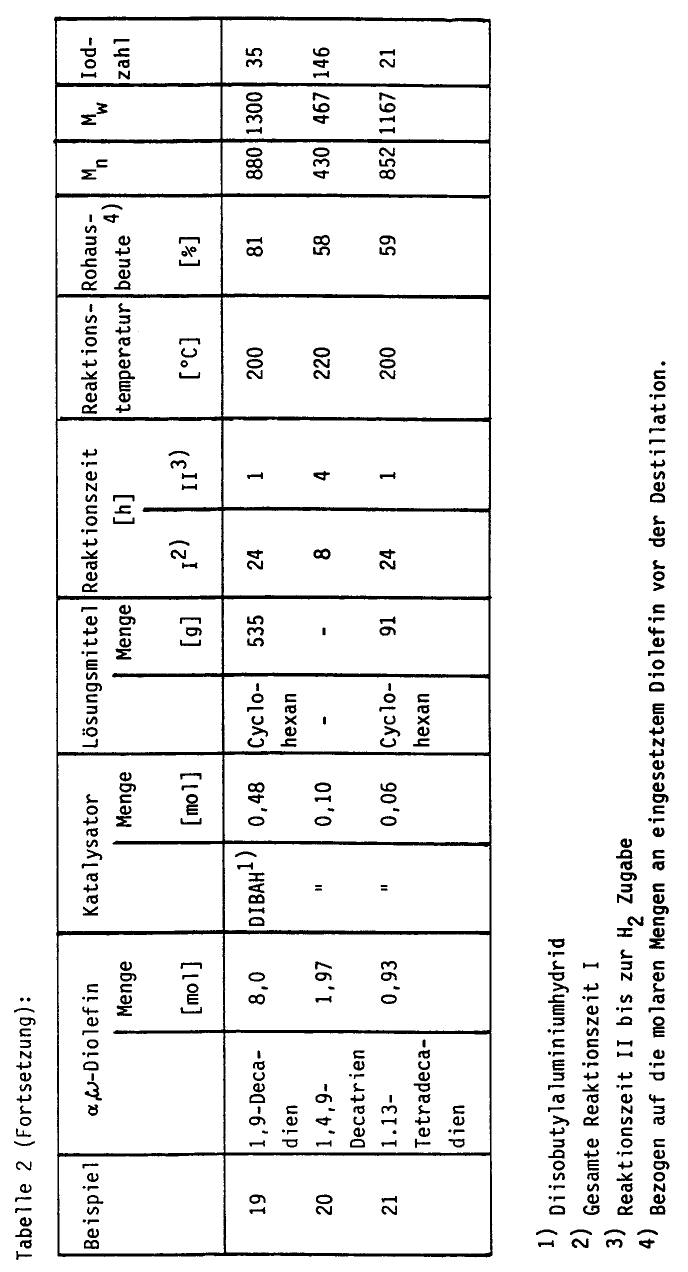

### Beispiele 22 - 25

Die in der Tabelle 3 angegebenen verschiedenen α,ω-Diolefine werden in der gleichen Apparatur und nach identischer Arbeitsweise wie in den Beispiel 1 bis 14 umgesetzt und, wie ebenfalls dort beschrieben, aufgearbeitet und charakterisiert. Alle weiteren Angaben über Ausgangs-, Endprodukte sowie Reaktionsbedingungen sind der Tabelle 3 zu entnehmen.

### Beispiele 26 - 27

Die in der Tabelle 4 angegebenen Ausgangsprodukte werden in Gegenwart eines Monoolefins in der gleichen Apparatur und nach identischer Arbeitsweise wie in den Beispiel 1 bis 14 umgesetzt und, wie ebenfalls dort beschrieben, aufgearbeitet und charakterisiert. Alle weiteren Angaben über Ausgangs-, Endprodukte sowie Reaktionsbedingungen sind der Tabelle 4 zu entnehmen.

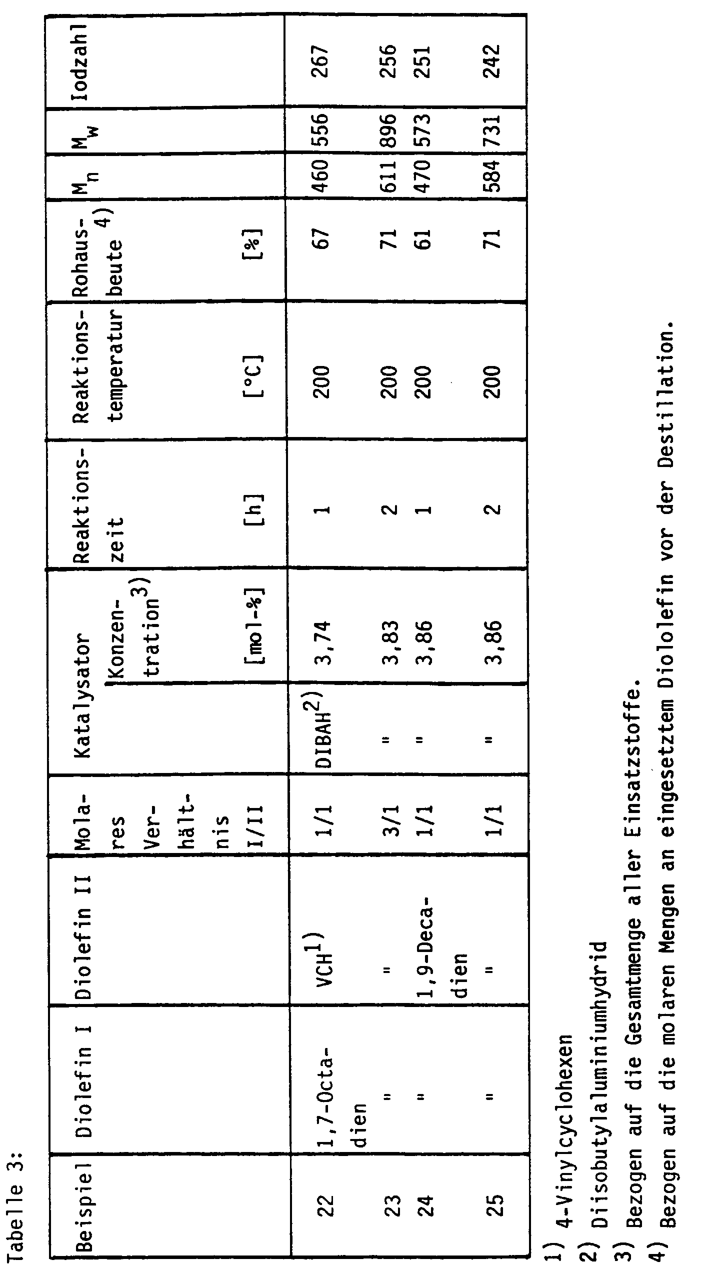

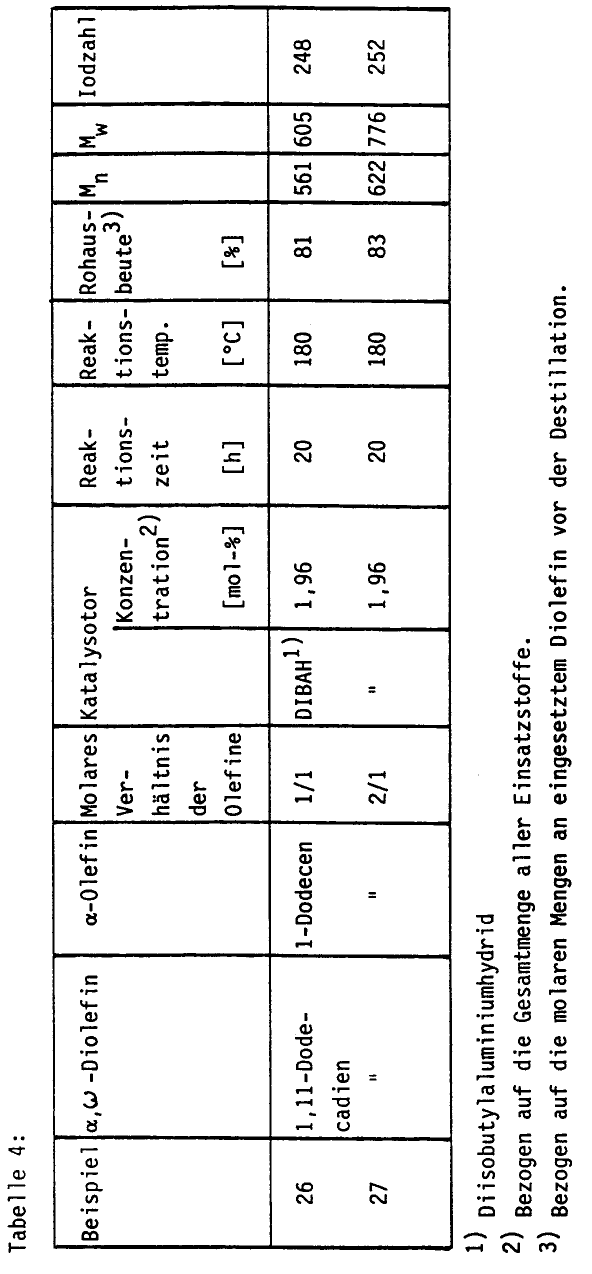

## Patentansprüche

1. Verfahren zur Herstellung von Methylidengruppen enthaltenden α,ω-ungesättigten Oligomeren aus α,ω-Diolefinen in Gegenwart von aluminiumorganischen Verbindungen als Katalysator,
dadurch gekennzeichnet,
daß die α,ω-Diolefine in flüssiger Phase bei einer Temperatur von 150 bis 350 °C zur Reaktion gebracht werden und die aluminiumorganischen Verbindungen der Formel AlX₃ oder AlX₂H entsprechen, wobei X einen aliphatischen, alicyclischen oder aromatischen Kohlenwasserstoffrest mit 1 bis 30 Kohlenstoffatomen darstellt.

2. Verfahren nach Anspruch 1, dadurch gekennzeichnet, daß die α, ω-Diolefine eine Kettenlänge von 5 oder 7 bis 25 Kohlenstoffatomen aufweisen und ausgenommen der endständigen Doppelbindungen durch Alkyl-, Aralkyl- und Cycloalkylgruppen substituiert sein können.

3. Verfahren nach den Ansprüchen 1 und 2,
dadurch gekennzeichnet,
daß die Konzentration der aluminiumorganischen Verbindungen 0,1 bis 10 Mol-%, bezogen auf die Menge an eingesetzten α,ω-Diolefinen, beträgt.

4. Verfahren nach den Ansprüchen 1 bis 3,
dadurch gekennzeichnet,
daß die Reaktion sowohl in Substanz oder in einem inerten Lösemittel durchgeführt wird.

5. Verfahren nach den Ansrpüchen 1 bis 4,
dadurch gekennzeichnet,
daß die Molmasse der Oligomeren durch Olefine mit nur einer endständigen Doppelbindung geregelt wird.

6. Verfahren nach den Ansprüchen 1 bis 4,
dadurch gekennzeichnet,
daß die Molmasse der Oligomeren durch Wasserstoff geregelt wird.

7. Verfahren nach den Ansprüchen 1 bis 6,
dadurch gekennzeichnet,
daß die α,ω-Diolefine weitere innenständige Doppelbindungen enthalten.

## Claims

1. A process for preparing methylidene group-containing, α,ω-unsaturated oligomers from α,ω-diolefins, in the presence of organoaluminium compounds acting as a catalyst, characterised in that the α,ω-diolefins are reacted in the liquid phase at a temperature of 150 to 350°C, and the organoaluminium compounds correspond to formula AlX₃ or AlX₂H, wherein X is an aliphatic, alicyclic or aromatic hydrocarbon group having 1 to 30 carbon atoms.

2. A process according to claim 1, characterised in that the α,ω-diolefins have a chain length of 5 or 7 to 25 carbon atoms and, with the exception of the terminal double bonds, may be substituted by alkyl, aralkyl and cycloalkyl groups.

3. A process according to claims 1 and 2, characterised in that the concentration of the organoaluminium compounds is 0.1 to 10 mol% based on the amount of α,ω-diolefins used.

4. A process according to claims 1 to 3, characterised in that the reaction is carried out either in substance or in an inert solvent.

5. A process according to claims 1 to 4, characterised in that the molar mass of the oligomers is controlled by olefins that have only one terminal double bond.

6. A process according to claims 1 to 4, characterised in that the molar mass of the oligomers is controlled by hydrogen.

7. A process according to claims 1 to 6, characterised in that the α,ω-diolefins comprise further non-terminal double bonds.

## Revendications

1. Procédé de fabrication d'oligomères α,ω-insaturés contenant des groupements méthylidène à partir de α,ω-dioléfines en présence de composés organoaluminiques comme catalysateur, caractérisé en ce que, les α,ω-dioléfines sont mises en réaction en phase liquide à une température de 150 à 350°C et les composés organoaluminiques correspondent à la formule AlX₃ ou AlX₂H, où X représente un reste hydrocarboné aliphatique, alicylique ou aromatique à 1-30 atomes de carbone.

2. Procédé selon la revendication 1, caractérisé en ce que les α,ω-dioléfines présentent une longueur de chaîne de 5 ou 7 à 25 atomes de carbone et, à l'exception des doubles liaisons terminales, peuvent être substituées par des groupements alkyle, aralkyle et cycloalkyle.

3. Procédé selon l'une des revendications 1 et 2, caractérisé en ce que, la concentration des composés organoaluminiques s'élève à 0,1-10 % en moles, par rapport à la quantité de α,ω-dioléfines mise en réaction.

4. Procédé selon l'une des revendications 1 à 3, caractérisé en ce que la réaction est conduite aussi bien dans la masse ou dans un solvant inerte.

5. Procédé selon l'une des revendications 1 à 4, caractérisé en ce que la masse molaire des oligomères est régulée au moyen d'oléfines n'ayant qu'une double liaison terminale.

6. Procédé selon l'une des revendications 1 à 4, caractérisé en ce que la masse molaire des oligoméres est régulée au moyen d'eau.

7. Procédé selon l'une des revendications 1 à 6, caractérisé en ce que, les α,ω-dioléfines contiennent d'autres doubles liaisons internes.
